# EUROPEAN PATENT APPLICATION

(11) **EP 1 790 317 A2**
(43) Date of publication of application: **30.05.2007**
(21) Application number: 06020303.1
(22) Date of filing: 27.09.2006
(51) Int. Cl.: A61F 2/16

(54) **Intraocular lens injecting system**

(30) Priority: 29.09.2005 JP 2005285538
(71) Applicant: NIDEK CO., LTD, Hiroishi-cho, Gamagori-shi Aichi-ken 443-0038 (JP)
(72) Inventor: Nagasaka, Shinji, Hiroishi-cho Gamagori-shi Aichi (JP)
(74) Representative: Schaeberle, Steffen

(57) **Abstract**

An intraocular lens injecting system includes: an injector for injecting a foldable intraocular lens in an eye; and a holder for holding the injector. The holder includes: a holding part for detachably holding the injector; and a restrictor for restricting movement of the intraocular lens placed in the injector held by the holding parts. The restrictor releases the restriction on the movement of the intraocular lens when the injector is detached from the holding part.

## Description

### BACKGROUND OF THE INVENTION

### (1) Field of the Invention

The present invention relates to an intraocular lens injecting system that injects an intraocular lens into an eye.

### (2) Related Art

As a type of operation methods for a cataract, a generally used operation is to extract a crystalline lens that has been clouded from the eye and then inject an intraocular lens in the eye as a substitute for the crystalline lens. In injecting the intraocular lens into an eye, an operator first makes an incision on the eye and then crushes and sucks the clouded crystalline lens through the incision using an ultrasonic cataract surgical apparatus- Subsequently, the intraocular lens is injected into a position where the crystalline lens was through the incision.

As an injector used to inject an intraocular lens into an eye, an injector having a mechanism that prevents an intraocular lens from moving inside in not use, such as on delivery, and allows it to move inside in use (injection) is known in the art (see US 2001/0007942A1 (JP-A-2001-104347)). This type of injector is held by (accommodated in) a holder (or received in a casing) on delivery to prevent damage. Therefore, to use the injector, a user has to release the injector from the holder, thereafter, make a non-movable intraocular lens movable.

### SUMMARY OF THE INVENTION

It is an object of the present invention to provide an intraocular lens injecting system that is capable of preparing an intraocular lens for use with ease by a simple configuration.

In order to solve the above problem, the present invention is characterized by having the following arrangement.
(1) An intraocular lens injecting system (100, 110, 120), comprising:
   an injector (50, 60) for injecting a foldable intraocular lens (1) in an eye; and
   a holder (30, 40, 70) for holding the injector, wherein the holder includes:
      a holding part (33a, 33b, 43a, 43b, 73a, 73b) for detachably holding the injector; and
      a restrictor (34, 44, 74) for restricting movement of the intraocular lens placed in the injector held by the holding part, wherein the restrictor releases the restriction on the movement of the intraocular lens when the injector is detached from the holding part.
(2) The intraocular lens inserting system according to (1), wherein the restrictor holds the intraocular lens in the injector when the injector is held by the holding part and the restrictor releases the intraocular lens when the injector is detached from the holding part.
(3) The intraocular lens inserting system according to (1), wherein
   the injector includes:
   an inserting portion (11) that includes an injecting port (lla) and is inserted through an incision made on the eye; and
   a lens place portion (12) where the intraocular lens is placed and which is formed on an opposite side to the injecting port of the inserting portion, and
   the restrictor restricts the movement of the intraocular lens placed in the lens place portion of the injector held by the holding part.
(4) The intraocular lens inserting system according to (3), wherein the restrictor holds the intraocular lens in the lens place portion when the injector is held by the holding part and the restrictor releases the intraocular lens when the injector is detached from the holding part.
(5) The intraocular lens inserting system according to (4), wherein the lens place portion can be opened and closed, and the restrictor holds the intraocular lens in a state in which the lens place portion is opened.
(6) The intraocular lens inserting system according to (5), wherein the restrictor restricts the movement of the intraocular lens in a perpendicular direction to an opening/closing direction of the lens place portion.
(7) The intraocular lens inserting system according to (6), wherein
   a length of the restrictor in the opening/closing direction is smaller than a diameter of an optical portion of the intraocular lens to be held by the restrictor, and
   a length of the restrictor in a perpendicular direction to the opening/closing direction is substantially same as the diameter of the optical portion of the intraocular lens to be held by the restrictor.

### BRIEF DESCRIPTION OF THE DRAWINGS

Fig. 1 is a schematic view showing an intraocular lens injecting system according to an embodiment of the invention;
Fig. 2 is a schematic view showing an intraocular lens;
Fig. 3 is a schematic view showing a lens cartridge of an injector;
Figs. 4A and 4B are schematic views showing a handpiece of the injector;
Figs. 5A and 5B are schematic views showing a holder that holds the injector;
Figs. 6A and 6B are views showing a state in which the injector is held by the holder;
Fig. 7 is a view showing a state in which the intraocular lens is disposed in the injector (the lens cartridge) held by the holder;
Figs. 8A and 8B are schematic views showing a modification of the holder;
Figs. 9A and 9B are views showing a state in which the injector is held by the modified holder; and
Figs. 10A and 10B are views showing a state in which a modified injector is held by the modified holder.

### DESCRIPTION OF PREFERRED EMBODIMENTS

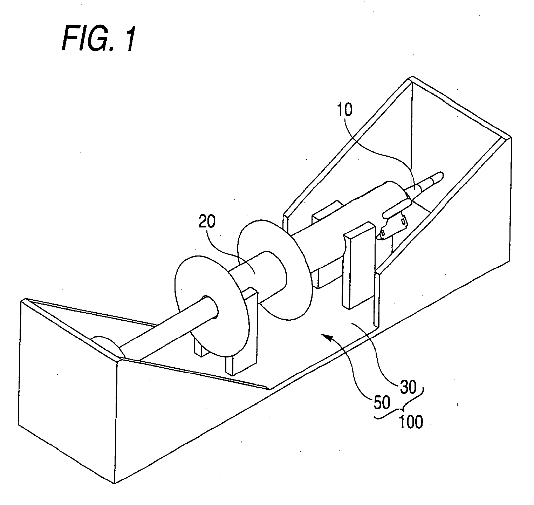

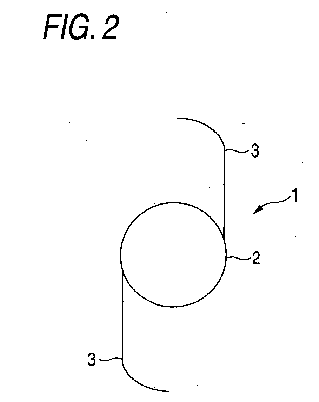

Preferred embodiments of the invention will be described hereafter with reference to accompanying drawings. Fig. 1 is a schematic view showing an intraocular lens injecting system according to an embodiment of the invention. Fig. 2 is a schematic view showing an intraocular lens. An intraocular lens injecting system 100 includes an injector 50 that injects a foldable intraocular lens 1 into an eye, and a holder (a casing) 30 that fixedly holds the injector 50. The injector 50 includes a lens cartridge (a lens holding unit) 10 that holds the intraocular lens 1 and a headpiece (cartridge holding unit) 20 that holds the cartridge 10.

The intraocular lens 1 includes an optical portion 2 having predetermined refractive power and a supporting portion 3 that supports the optical portion 2 in an eye. The optical portion 2 is formed of an existing material that is used for a foldable optical portion of an intraocular lens, such as, HEMA (Hydroxyethylmethacrylate), a composite of acrylic acid ester and methacrylate, etc. Further, the supporting portion 3 is formed of an existing material that is used for a supporting portion of an intraocular lens, such as PMMA (Polymethylmethacrylate) etc. The shape of the intraocular lens 1 is not limited to those in the embodiments of the invention and any shape may be applied as long as it is foldable.

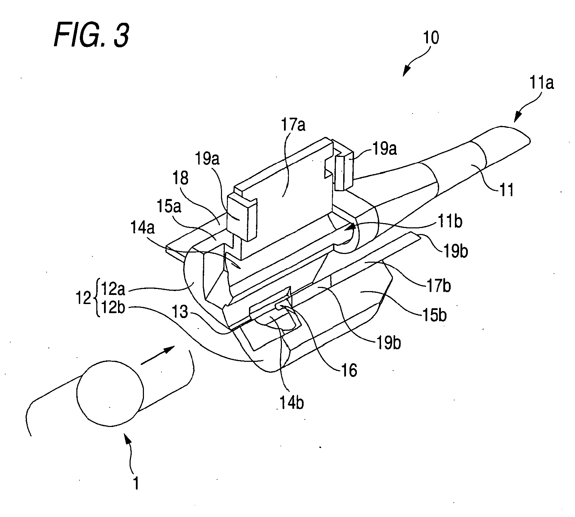

Fig. 3 is a schematic view of the cartridge 10. The cartridge 10 includes an inserting portion 11 that is to be inserted through an incision on an eye and a lens place portion 12 where the intraocular lens 1 is to be placed (held). The inserting portion 11 is a tube-shaped part tapered at the front end, in which the intraocular lens 1 folded at the lens place portion 12 passes through a hollow portion and is folded smaller, and then extruded out (discharged outside) through an exit (an injecting port) 11a. The lens place portion 12 includes a fixed part 12a that is fixed to the inserting portion 11 and a movable part (an opening/closing part) 12b that is coupled to the fixed part 12a through a hinge 13. The fixed part 12a includes a placing surface 14a and the movable part 12b includes a placing surface 14b. The intraocular lens 1 is placed on the placing surfaces 14a and 14b in a state that the lens place portion 12 (the fixed part 12a and the movable part 12b) is opened. The intraocular lens 1 placed in the state that the lens place portion 12 is opened is not folded (but, movement in the opening/closing direction of the lens place portion 12 is restricted). When the lens place portion 12 (the fixed part 12a and the movable part 12b) is closed after the intraocular lens 1 is placed, the placing surfaces 14a and 14b form the same shape (half circle) as an inlet 11b of the inserting portion 11 and the intraocular lens 1 placed on the placing surfaces 14a and 14b is folded in the same shape. In other word, the placing surfaces 14a and 14b are curved such that the intraocular lens 1 is not folded when the lens place portion 12 is opened, and it is folded when the lens place portion 12 is closed.
The fixed part 12a is provided with a cover 15a and the movable part 12b is provided with a cover 15b. The covers 15a and 15b cover the upside of the placing surfaces 14a and 14b when the lens place portion 12 is closed. A convex portion 16 extending down toward the placing surfaces 14a and 14b is formed at the side of the cover 15b facing the cover 15a. The convex portion 16 prevents the intraocular lens 1 from being folded in directions, not the appropriate folding direction of the intraocular lens 1, i.e. not the opening/closing direction of the lens place portion 12.
Further, a joint plate portion 17a extending upward is provided at the side of the cover 15a facing the cover 15b and a joint plate portion 17b extending upward is provided at the side of the cover 15b facing the cover 15a. The joint plate portion 17a is provided with a male hook 19a that is snap-fitted with a female hook 19b provided to the joint plate portion 17b. When the lens place portion 12 is closed, the joint plate portion 17a is jointed with the joint plate portion 17b and the male hook 19a is snap-fitted with the female hook 19b.

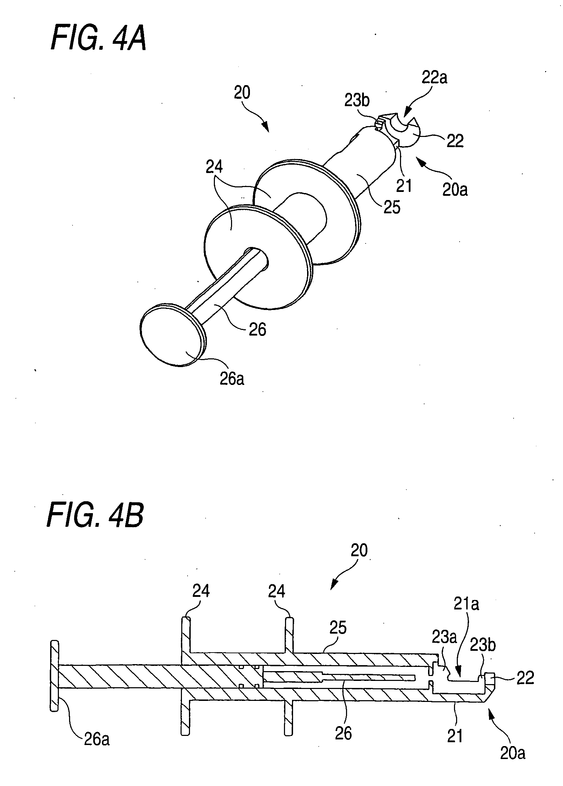

Figs. 4A and 4B are schematic views of the handpiece 20. Fig. 4A is a perspective view and Fig. 4B is a cross-sectional view. The handpiece 20 includes a cylinder (a body unit) 25 where the cartridge 10 is mounted and a piston (a lens extruding unit) 26 that is inserted in the cylinder 25. A mounting portion 20a for the cartridge 10 is formed at the front end of the cylinder 25. Further, a flare-shaped finger portion 24 where an user (an operator) puts his/her fingers to hold the handpiece 20 (the cylinder 25) such as a syringe is formed on the outer periphery of the cylinder 25. A pushing portion 26a for the user to push the piston 26 into the cylinder 25 is provided at the back end of the piston 26.

The mounting portion 20a includes a first receiving portion 21 for the lens place portion 12 (the fixed part 12a) of the cartridge 10 to be placed (fitted) and a second receiving portion 22 for the inserting portion 11 of the cartridge 10 to be placed (fitted). The first receiving portion 21 is shaped like a quadrant circle at the end of the cylinder 25 and the second receiving portion 22 has a letter-C shape in which an arc thereof is a little longer than a half of the circumference around the center axis of the cylinder 25. When the cartridge 10 is mounted on the mounting portion 20a, the bottom of the fixed part 12a contacts with the first receiving portion 21 and the bottom of the inserting portion 11 contacts with the second receiving portion 22. A recess 21a is formed on the first receiving portion 21 by two convex portions 23a and 23b and a concave portion 18 (see Fig. 3) of the cartridge 10 is fitted in the recess 21a. Further, the inserting portion 11 is fitted in a recess 22a of the second receiving portion 22. As combined as described above, the cartridge 10 is fixedly held at the mounting portion 20a in the state that the lens place portion 23 is opened.

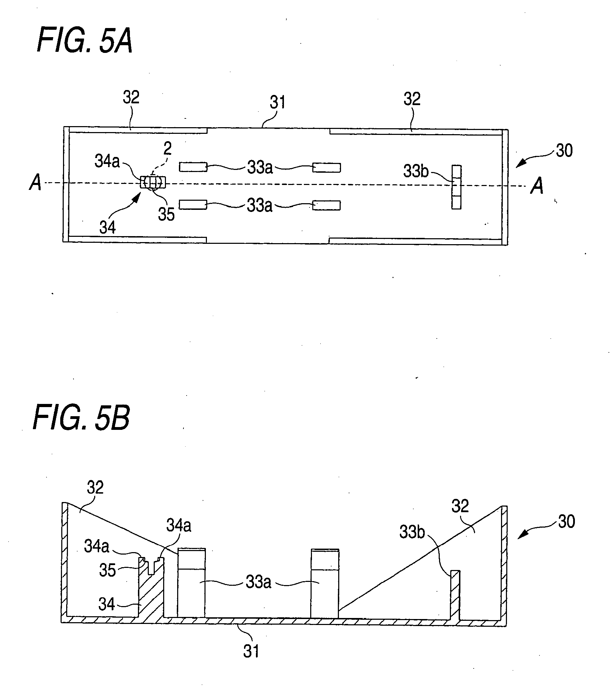

Figs. 5A and 5B are schematic views of the holder 30, Fig. 5A is a plan cross-sectional view taken from above and Fig. 5B is a cross-sectional view taken along a line A-A of Fig. 5A. A base 31 is provided with a wall 32 (a fence) along its edge and includes holding parts 33a and 33b for holding the injector 50. Two holding parts 33a are provided so as to chuck the cylinder 25 and the holding part 33b supports the piston 26 from underneath.
A circular arc spot facing for the cylinder 25 fitted is formed at the upside of the holding part 33a and another circular arc spot facing for the piston fitted is formed at the upside of the holding part 33b.
The base 31 is provided with a restrictor 34 that restricts movement of the intraocular lens 1 in a forward/backward direction of the injector 50 (a perpendicular direction to the opening/closing direction of the lens place portion 12) in the lens place portion 12 of the cartridge 10. The restrictor 34 extends in the direction that the injector 50 is detached from the holder 30. A cavity 35 where the intraocular lens 1 (the optical portion 2) is placed is formed on upside of the restrictor 34. The front and back sidewalls 34a of the cavity 35 are curved to fit with the circular optical portion 2 and chuck the optical portion 2 from front and rear when the optical portion 2 is placed in the cavity 35. Further, the bottom of the cavity 35 is more curved than the optical portion 2, so that when the optical portion 2 is placed in the cavity 35, the periphery of the optical portion 2 contacts with the edge inside the cavity 35. Accordingly, the intraocular lens 1 is stably placed (held).

Meanwhile, the width of the restrictor 34 in a left/right direction of the injector 50 (the holder 30) (the opening/closing direction of the lens place portion 12) is smaller than the diameter of the optical portion 2, so that a part of the edge of the optical portion 2 protrudes to the left and right of the cavity 35.

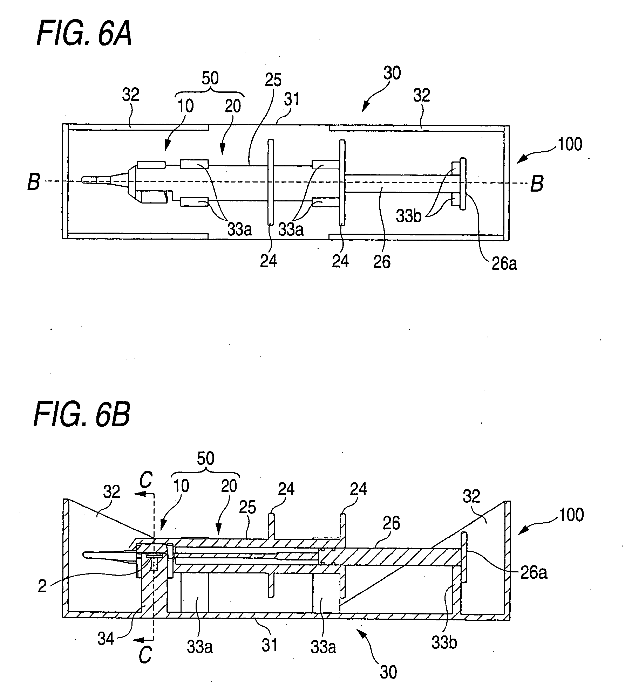

Figs. 6A and 6B are views showing a state in which the injector 50 is held by the holder 30 (i.e. an intraocular lens injecting system 100). Fig. 6A is a plan view taken from above and Fig. 6B is a cross-sectional view taken along a line B-B of Fig. 6a. The lens place portion 12 of the cartridge 10 is opened and the intraocular lens 1 placed in the cavity 35 of the restrictor 34 is covered by the cartridge 10. On the other hand, movement of the piston 26 in the axial direction thereof (the forward/backward direction) is restricted by the holding part 33b. In the above state, because the front end of the piston 26 is not inserted in the cartridge 10, the intraocular lens 1 is not extruded out by the piston 26.

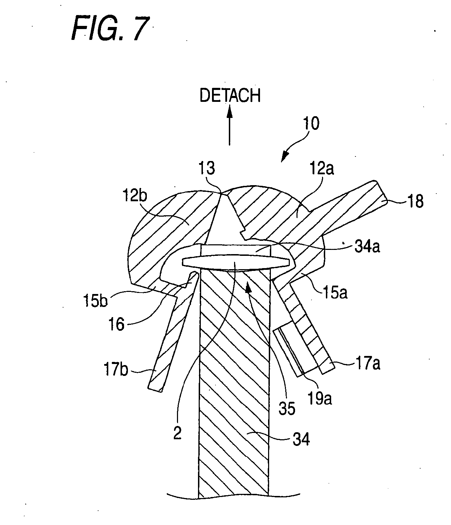

Fig. 7 is a cross-sectional view taken along a line C-C of Fig. 6, showing a state in which the intraocular lens 1 is placed in the injector 50 (the cartridge 10) held by the holder 30. The intraocular lens 1 is placed on the restrictor 34 and covered by the cartridge 10 such that it does not contact with the placing surfaces 14a and 14b in the lens place portion 12- As described above, because the width in the left/right direction of the restrictor 34 is smaller than the diameter of the optical portion 2 and a part of the edge of the optical portion 2 protrudes to the left and right of the restrictor 34, when the injector 50 is detached from the holder 30 in the direction of an arrow, the intraocular lens 1 is caught by the placing surfaces 14a and 14b of the cartridge 10 and remains in the lens place portion 12.

The operation of the above described injection system 100 will be described hereafter.
First, the user takes the injecting system 100 out from a case (not shown) for keeping an aseptic condition, thereafter, detaches the injector 50 from the holder 30. In the detaching of the injector 50 as described above, the cartridge 10 is moved in the direction of arrow in Fig. 7 (i.e. upward from the holder 30), and because movement of the intraocular lens 1 placed on the restrictor 34 in the detaching direction is not restricted, the intraocular lens 1 is lifted, contacting with the placing surfaces 14a and 14b. As a result, the intraocular lens 1 is detached from the restrictor 34 (the cavity 35) (i.e. released from the restrictor 34) and remains in the cartridge 10 (the lens place portion 12).

After the injector 50 is detached from the holder 30, the user injects a viscoelastic substance into the cartridge 10 and closes the joint plate portion 17a and 17b (i.e. locks the male and female hooks 19a and 19b). As a result, the intraocular lens l.is folded with taking the shape of the placing surfaces 14a and 14b. After locking, as the user pushes the pushing portion 26a to move the piston 26 forward, the piston 26 moves toward the front end in the cylinder 25 and inserts the intraocular lens 1 from the lens place portion 12 into the inserting portion 11. As the intraocular lens 1 is inserted into the inserting portion 11 further, the intraocular lens 1 is folded smaller (wounded) and then the small folded intraocular lens 1 is discharged out of the exit 11a at the front end of the inserting portion 11.

As described above, while the injector 50 is detached from the holder 30, movement of the intraocular lens 1 in the cartridge 10 is released, thus saving the user's effort.

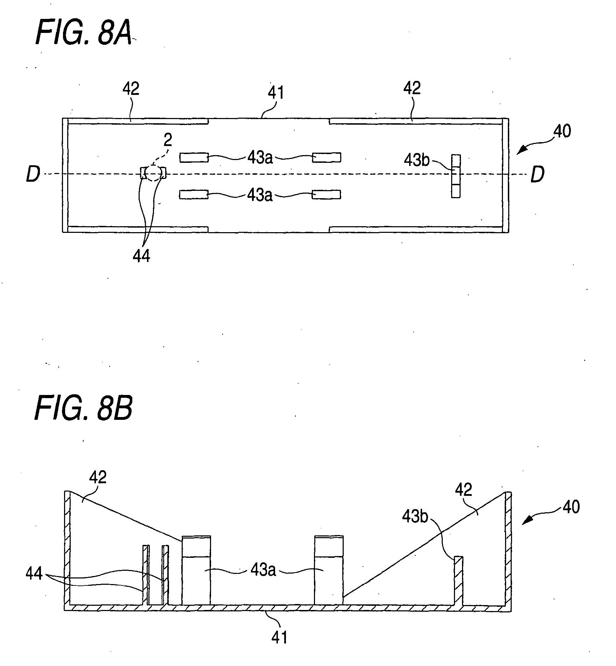

Figs. 8A and 8B arc schematic views showing a modification of the holder. Fig. 8A is a plan view taken from above and Fig. 8B is a cross-sectional view taken along a line D-D of Fig. 8A. A modified holder 40 includes a base 41, a wall (a fence) 42, holding parts 43a and 43b, and a restrictor 44. The base 41, the wall 42, the holding parts 43a and 43b are the same as the base 31, the wall 32, and the holding parts 33a and 33b, respectively. The restrictor 44 has a configuration that the sidewalls 34a of the restrictor 34 extends to the base 41 and chucks the intraocular lens 1 by the curved surface that is formed so as to fit with the diameter of the optical portion 2.

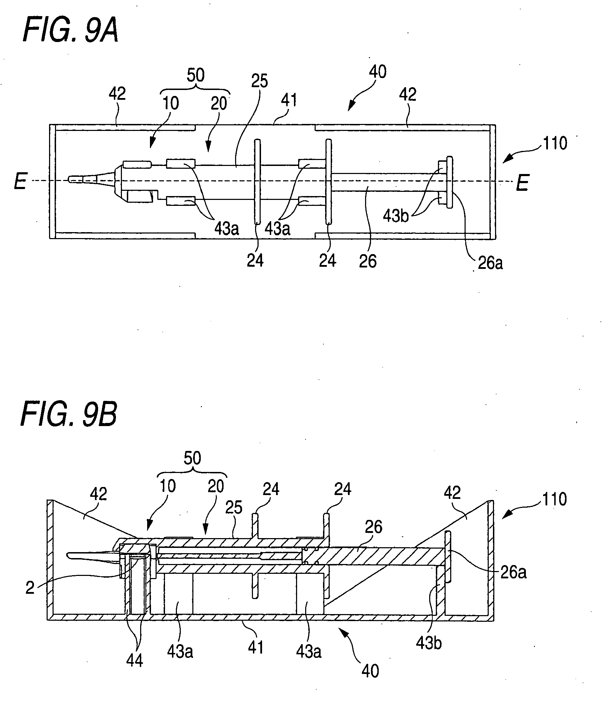

Figs. 9A and 9B are views showing a state in which the injector 50 is held by the holder 40 (i.e. an intraocular lens injecting system 110). Fig. 9A is a plan view taken from above and Fig. 9B is a cross-sectional view taken along a line E-E of Fig. 9A. Forward/backward movement of the intraocular lens 1 is restricted since the optical portion 2 is chucked by the restrictor 44.
Because the restrictor 44 does not support the downside of the optical portion 2, the optical portion 2 is supported through contact with the inside wall (the placing surfaces 14a and 14b) of the cartridge 10.

As for the above embodiments, the lens place portion 12 of the cartridge 10 opens and closes and the restrictor 34 or 44 restricts movement of the intraocular lens 1 in the lens place portion 12 open, but the invention is not limited thereto.

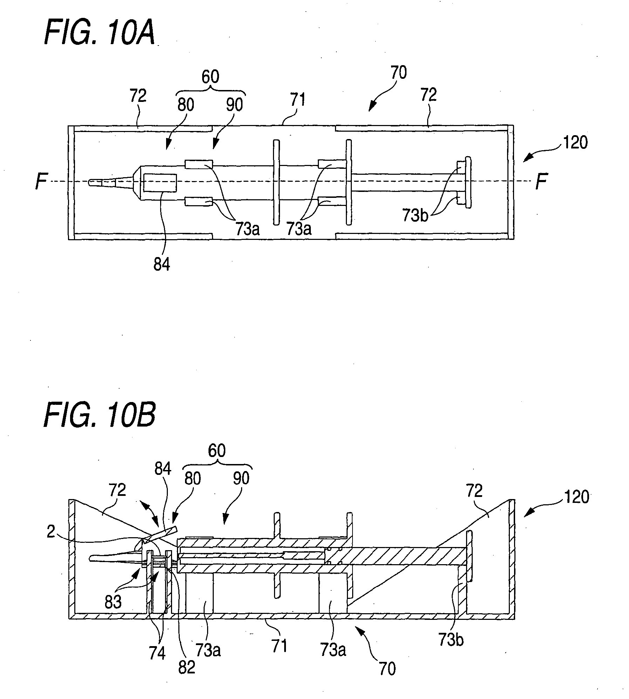

Figs. 10A and 10C are views showing a state in which an injector 60 is held by a holder 70 (i.e. an intraocular lens injecting system 120). Fig. 10A is a plan view taken from above and Fig. 10B is a cross-sectional view taken along a line F-F of Fig. 10A. The injector 60 includes a lens cartridge (a lens holding unit) 80 and a handpiece (a cartridge holding unit) 90 where the cartridge 80 is mounted. Two holes 83, through which a restrictor 74 is inserted, are formed through the bottom of a lens place portion 82 of the cartridge 80. Further, a cover 84 is provided at the upside of the lens place portion 82 and the intraocular lens 1 is placed in the lens place portion 82 when the cover 84 is opened.
The holder 70 includes a base 71, a wall (a fence) 72, holding parts 73a and 73b, and the restrictor 74. The base 71, the wall 72, and the holding parts 73a and 73b are the same as the base 31, wall 32, and the holding parts 33a and 33b, respectively. In addition, the restrictor 74 is the same as the restrictor 44.

The operation having the aforementioned configuration is described hereafter.
First, the injector 60 is constructed by mounting the cartridge 80 to the handpiece 90 and the assembly is mounted to the holder 70 for holding. At this time, the restrictor 74 is inserted through the holes 83 and finally into the cartridge 80 (the lens place portion 82). Subsequently, the cover 84 is opened and the intraocular lens 1 is interposed by the restrictor 74 in the cartridge 80 (the lens place portion 82), and then the cover 84 is closed.

When the user uses the injector 60, the user detaches the injector 60 from the holder 70. In this detachment, the restrictor 74 is pulled out of the cartridge 80 and the intraocular lens 1 held on the restrictor 74 remains in the lens place portion 82- As a result, restriction on movement of the intraocular lens 1 is released and the intraocular lens 1 can be discharged from the injector 60.

Meanwhile, it is preferable to size the holes 83 such that the intraocular lens 1 does not fall from the cartridge 80 to such an extent that the holes 83 does not affect to the discharging of the intraocular lens 1.

As for the embodiments above, the lens cartridge and the handpiece are integrally formed, but not limited thereto, and only the lens cartridge may be held to the holder. According to this configuration, the lens cartridge is held by the holding parts of the holder and movement of the intraocular lens in the lens cartridge is restricted by the restrictor of the holder.

## Claims

1. An intraocular lens injecting system (100, 110, 120), comprising:
an injector (50, 60) for injecting a foldable intraocular lens (1) in an eye; and
a holder (30, 40, 70) for holding the injector, wherein the holder includes:
a holding part (33a, 33b, 43a, 43b, 73a, 73b) for detachably holding the injector; and
a restrictor (34, 44, 74) for restricting movement of the intraocular lens placed in the injector held by the holding part, wherein the restrictor releases the restriction on the movement of the intraocular lens when the injector is detached from the holding part.

2. The intraocular lens inserting system according to claim 1, wherein the restrictor holds the intraocular lens in the injector when the injector is held by the holding part and the restrictor releases the intraocular lens when the injector is detached from the holding parts.

3. The intraocular lens inserting system according to claim 1, wherein
the injector includes:
an inserting portion (11) that includes an injecting port (11a) and is inserted through an incision made on an eye; and
a lens place portion (12) where the intraocular lens is placed and which is formed on an opposite side to the injecting port of the inserting portion, and
the restrictor restricts the movement of the intraocular lens placed in the lens place portion of the injector held by the holding part.

4. The intraocular lens inserting system according to claim 3, wherein the restrictor holds the intraocular lens in the lens place portion when the injector is held by the holding part and the restrictor releases the intraocular lens when the injector is detached from the holding part.

5. The intraocular lens inserting system according to claim 4, wherein the lens place portion can be opened and closed, and the restrictor holds the intraocular lens in a state in which the lens place portion is opened.

6. The intraocular lens inserting system according to claim 5, wherein the restrictor restricts the movement of the intraocular lens in a perpendicular direction to an opening/closing direction of the lens place portion.

7. The intraocular lens inserting system according to claim 6, wherein
a length of the restrictor in the opening/closing direction is smaller than a diameter of an optical portion of the intraocular lens to be held by the restrictor, and
a length of the restrictor in a perpendicular direction to the opening/closing direction is substantially same as the diameter of the optical portion of the intraocular lens to be held by the restrictor.
